# EUROPÄISCHE PATENTSCHRIFT

(11) **EP 1 270 576 B1**
(45) Veröffentlichungstag und Bekanntmachung des Hinweises auf die Patenterteilung: **15.09.2004**
(21) Anmeldenummer: 02013861.6
(22) Anmeldetag: 22.06.2002
(51) Int. Cl.: C07D 471/08, A61K 31/439, A61P 9/00, C07D 471/10

(54) **3-Phenyl-3,7-diazabicyclo(3,3,1)nonan-Verbindungen sowie Verfahren zu ihrer Herstellung und diese Verbindungen enthaltende Arzneimittel**
3-phenyl-3,7-diazabicyclo[3,3,1]nonanes, process for their preparation and pharmaceutical compositions containing them
3-Phenyl-3,7-diazabicyclo[3,3,1]nonanes, procédé pour leur préparation et compositions pharmaceutiques les contenant

(30) Priorität: 28.06.2001 DE 10131217
(43) Veröffentlichungstag der Anmeldung: 02.01.2003
(73) Patentinhaber: Solvay Pharmaceuticals GmbH, 30173 Hannover (DE)
(72) Erfinder: Schön, Uwe, 31303 Burgdorf (DE); Messinger, Josef, 31319 Sehnde (DE); Brückner, Reinhard, 30559 Hannover (DE); Ziegler, Dieter, 30966 Hemmingen (DE)
(74) Vertreter: Bauriegel, Lutz

(56) Entgegenhaltungen:
- EP-A- 0 103 833
- EP-A- 0 306 871

## Beschreibung

Die vorliegende Erfindung betrifft neue 3,7,9,9-tetrasubstituierte 3,7-Diazabicyclo[3,3,1]nonan-Verbindungen, welche in 3-Stellung einen substituierten Phenylrest tragen, und deren Salze sowie diese Verbindungen enthaltende pharmazeutische Zubereitungen und Verfahren zur Herstellung dieser Verbindungen.

Aus der EP-A 0 665 014 sind bereits 3-Benzoyl-3,7-diazabicyclo[3,3,1]nonan-Derivate mit antiarrhythmischen Eigenschaften bekannt.

Aus der EP-A 0 665 228 sind ferner 3-Phenylsulfonyl-3,7-diazabicyclo[3,3,1]nonan-Derivate sowie diese enthaltende Arzneimittel mit antiarrhythmischen Eigenschaften bekannt.

Der Erfindung liegt die Aufgabe zugrunde, neue antiarrhythmisch wirksame Wirkstoffe mit verbessertem Wirkungsprofil zu entwickeln. Ferner liegt der Erfindung die Aufgabe zugrunde, neue 3,7-Diazabicyclo[3,3,1]nonan-Verbindungen mit wertvollen pharmakologischen Eigenschaften zu entwickeln.

Es wurde nun gefunden, daß die neuen, in 3-Stellung einen substituierten Phenylrest tragenden, 3,7,9,9-tetrasubstituierten 3,7-Diazabicyclo[3,3,1]nonan-Verbindungen wertvolle pharmakologische Eigenschaften zur Behandlung und/oder Prophylaxe von Herzrhythmusstörungen besitzen und ein antiarrhythmisches Wirkprofil zeigen, welches sie zur Behandlung von Herzrhythmusstörungen, insbesondere tachycarden Arrhythmien, besonders geeignet macht.

Die Erfindung betrifft daher neue Verbindungen der allgemeinen Formel I 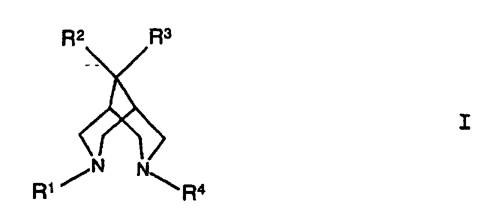 worin
- R¹: eine Alkylgruppe mit 1 - 6 Kohlenstoffatomen oder eine Cycloalkylalkylgruppe mit 4 - 7 Kohlenstoffatomen bedeutet,
- R²: niederes Alkyl bedeutet und
- R³: niederes Alkyl bedeutet oder
- R² und R³: gemeinsam eine Alkylenkette mit 3 - 6 Kohlenstoffatomen bilden,
- R⁴: für einen in ortho- oder para-Stellung durch Nitro, Cyano oder niederes Alkanoyl monosubstituierten oder in ortho- und para-Stellung durch Nitro disubstituierten Phenylrest steht, und
deren physiologisch verträgliche Säureadditionssalze.

Sofern in den Verbindungen der Formel I R¹ für eine Alkylgruppe steht, kann diese geradkettig oder verzweigt sein und 1 - 6, vorzugsweise 3 - 5, insbesondere 4 Kohlenstoffatome enthalten. Eine Cycloalkylalkylgruppe R¹ kann vorzugsweise Cyclopropylmethyl bedeuten. Als besonders geeignete Reste R¹ haben sich Alkylreste mit 3 - 5 Kohlenstoffatomen erwiesen.

Sofern die Substituenten R² und R³ niederes Alkyl darstellen, können diese Alkylgruppen geradkettig oder verzweigt sein und 1 - 4, vorzugsweise 1 - 3 Kohlenstoffatome enthalten und insbesondere Methyl bedeuten. Sofern R² und R³ gemeinsam eine Alkylengruppe bilden, kann diese 3 - 6, vorzugsweise 4 - 5, Kohlenstoffatome enthalten. Insbesondere haben sich solche Verbindungen, worin R² und R³ gemeinsam eine Alkylenkette mit 4 - 5 Kohlenstoffatomen bedeuten, als geeignet erwiesen.

Der Substituent R⁴ stellt eine substituierte Phenylgruppe dar, worin die Substituenten der Phenylgruppe in ortho- oder para-Stellung angeordnet sind. Ein niederer Alkanoyl-Substituent kann 2 - 5, vorzugsweise 2 - 3, Kohlenstoffatome enthalten. Vorzugsweise stellt R⁴ eine in para-Stellung durch Cyano oder niederes Alkanoyl, insbesondere Cyano, substituierte Phenylgruppe dar.

Erfindungsgemäß werden die neuen Verbindungen der Formel I und deren Säureadditionssalze erhalten, indem man in an sich bekannter Weise Verbindungen der allgemeinen Formel II 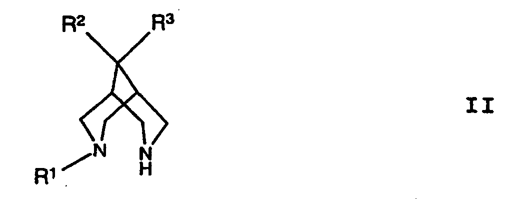 worin R¹, R² und R³ obige Bedeutung besitzen, mit Verbindungen der allgemeinen Formel III

R⁴-X III

worin R⁴ obige Bedeutung besitzt und X Halogen bedeutet, umsetzt, und gegebenenfalls freie Verbindungen der Formel I in ihre Säureadditionssalze überführt oder die Säureadditionssalze in die freien Verbindungen der Formel I überführt.

Die Umsetzung der Verbindungen der Formel II mit Verbindungen der Formel III kann auf an sich bekannte Weise unter zur Substitution von aromatischen Halogeniden durch Amine üblichen Bedingungen erfolgen. Als Halogene in den Verbindungen der Formel II kommen insbesondere Chlor oder Fluor in Frage. Die Umsetzung wird in einem unter den Reaktionsbedingungen inerten organischen Lösungsmittel bei Temperaturen zwischen Raumtemperatur und Siedetemperatur des Reaktionsgemisches durchgeführt. Als organische Lösungsmittel eignen sich beispielsweise Äther, insbesondere cyclische Äther wie Tetrahydrofuran, niedere Alkanole wie Butanol, niedere aliphatische Ketone wie Aceton, Dimethylsulfoxid, Dimethylformamid, aromatische Kohlenwasserstoffe wie Benzol oder Toluol oder Gemische aus den vorgenannten Lösungsmitteln. Zweckmäßigerweise wird die Reaktion unter basischen Bedingungen z. B. in Gegenwart einer mindestens äquivalenten Menge einer Base durchgeführt. Beispiele geeigneter Basen sind anorganische Basen wie Alkalimetallhydroxide, Alkalimetallcarbonate, Alkalimetallamide oder Alkalimetallhydride oder organische Basen wie tertiäre Niederalkylamine.

Die Verbindungen der Formel I können auf an sich bekannte Weise aus dem Reaktionsgemisch isoliert und gereinigt werden. Säureadditionssalze können in üblicher Weise in die freien Basen überführt werden und diese können gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise deren übliche Salze mit anorganischen Säuren, z. B. Halogenwasserstoffsäuren, insbesondere Chlorwasserstoffsäure, Schwefelsäure oder Phosphorsäuren, oder mit organischen Säuren, beispielsweise niederen aliphatischen Mono-, Di- oder Tricarbonsäuren wie Maleinsäure, Fumarsäure, Milchsäure, Weinsäure, Essigsäure oder Zitronensäure, oder mit Sulfonsäuren, beispielsweise Niederalkansulfonsäuren wie Methansulfonsäure oder gegebenenfalls im Benzolring durch Halogen oder niederes Alkyl substituierte Benzolsulfonsäuren wie p-Toluolsulfonsäure.

Falls in den Verbindungen der Formel I die Substituenten R² und R³ verschieden sind, enthalten die Verbindungen ein Chiralitätszentrum und können in zwei optisch aktiven Formen oder als Racemat vorliegen. Die vorliegende Erfindung umfaßt sowohl die racemischen Gemische wie auch die optischen Isomeren dieser Verbindungen der Formel I. Die optisch aktiven Verbindungen können aus den racemischen Gemischen in an sich bekannter Weise durch übliche Trennverfahren erhalten werden, z. B. durch Chromatographie an chiralen Trennmaterialien oder fraktionierte Kristallisation geeigneter Salze mit optisch aktiven Säuren. Enantiomerenreine Verbindungen können auch durch Synthese aus entsprechenden enantiomerenreinen Ausgangsverbindungen der Formel II hergestellt werden.

Die Ausgangsverbindungen der Formel II sind aus der DE-OS 2658 558, der EP-A 0 103 833 und der EP-A 0 306 871 bekannt und/oder können nach den in diesen Schriften beschriebenen Methoden oder analog zu den in diesen Schriften beschriebenen Methoden auf an sich bekannte Weise hergestellt werden. Die Ausgangsverbindungen der Formel III sind bekannt und/oder können nach an sich bekannten Verfahren oder analog zu an sich bekannten Verfahren hergestellt werden.

Es wurde nun überraschenderweise gefunden, daß die erfindungsgemäßen Verbindungen der Formel I und ihre physiologisch verträglichen Säureadditionssalze besonders günstige antiarrhythmische Wirkungen besitzen. Sie zeigen insbesondere Klasse-III antiarrhythmische Eigenschaften, welche zu einer Verlängerung des QT-Intervalls im EKG führen und bewirken eine Verlängerung der effektiven Refraktärzeit im Herzen. Die Verbindungen besitzen ein günstiges Wirkprofil mit guter Verträglichkeit, langer Wirkungsdauer und einer so hohen Selektivität der antiarrhythmischen Wirkung gegenüber blutdrucksenkenden Eigenschaften, daß im antiarrhythmisch wirksamen Dosisbereich keine therapeutisch unerwünschte Beeinflussung des Blutdruckes auftritt. Die Verbindungen zeichnen sich dadurch aus, daß die antiarrhythmische Wirkung unter tachycarden Bedingungen besonders gut ausgeprägt ist. Ferner weisen die erfindungsgemäßen Verbindungen Eigenschaften auf, welche darauf schließen lassen, daß ihre antiarrhythmischen Eigenschaften mit überraschend geringen proarrhythmogenen Begleitwirkungen einhergehen.

Die antiarrhythmischen Wirkungen der Verbindungen lassen sich in pharmakologischen Standardtestmethoden nachweisen. Die bei den Testmethoden für die einzelnen Testsubstanzen angegebenen Beispielsnummern beziehen sich jeweils auf die nachstehenden Herstellungsbeispiele.

### Beschreibung der pharmakologischen Untersuchungsmethoden.

### 1. Bestimmung der minimalen toxischen Dosis

Männlichen Mäusen von 20 bis 25 g Gewicht werden p.o. Maximaldosen von 300 mg/kg der Testsubstanz verabreicht. Die Tiere werden 3 Stunden lang sorgfältig auf Toxizitätssymptome beobachtet. Über einen Zeitraum von 72 Stunden nach der Applikation werden zusätzlich alle Symptome und Todesfälle registriert. Begleitsymptome werden ebenfalls beobachtet und registriert. Wenn Tod oder starke toxische Symptome beobachtet werden, werden weiteren Mäusen zunehmend geringere Dosen verabreicht, bis keine toxischen Symptome mehr auftreten. Die niedrigste Dosis, welche Tod oder starke toxische Symptome hervorruft, wird in der nachfolgenden Tabelle A als minimale toxische Dosis angegeben.

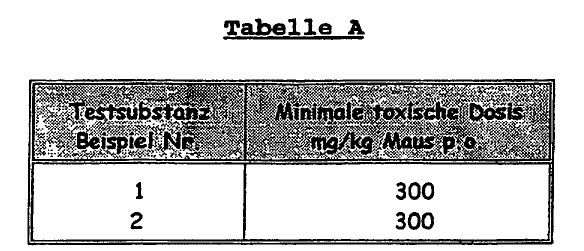

### 2. In vivo Untersuchung der antiarrhythmischen Eigenschaften der Substanzen unter tachycarden Bedingungen an narkotisierten Meerschweinchen.

Die Wirkungen der Substanzen auf die effektive Refraktärzeit (= ERP) und den Blutdruck bei i.v.-Applikation bei erhöhter Herzfrequenz wurden an narkotisierten Meerschweinchen untersucht. Den Tieren wurde unter Vollnarkose ein bipolarer Reizkatheter über eine Jugularvene in den rechten Ventrikel geschoben. Über diesen wurde die Herzfrequenz der Tiere durch elektrische Reizung während der gesamten Untersuchung auf ca. 150 % ihrer normalen Herzfrequenz gehalten. In die andere Jugularvene wurde eine Kanüle zur i.v.-Applikation der Testsubstanzen eingeführt. Während der Untersuchung wurden der systolische und der diastolische arterielle Blutdruck (= SAP und DAP) in einer Arteria carotis über einen Druckmesser (Statham-Drucktransducer) gemessen. Die Testsubstanzen wurden i. v. in steigenden Dosen (kumulativ) appliziert. Vor Applikation der ersten Dosis und jeweils 8 Minuten nach jeder Dosisgabe wurde die ERP mittels eines Doppelpulsprotokolls bestimmt. Die Dosis, bei der eine Verlängerung der ERP auf 115 % des Ausgangswertes erreicht wurde, wurde als effektive Dosis (= ERP-ED₁₁₅) berechnet. Als effektive Dosen für eine blutdrucksenkende Wirkung wurden die Dosis, bei der der SAP auf 85 % seines Ausgangswertes gesenkt wurde (= SAP-ED₈₅), und die Dosis, bei welcher der DAP auf 85 % seines Ausgangswertes gesenkt wurde (= DAP-ED₈₅), berechnet.

In der nachfolgenden Tabelle B werden die mit der vorstehend beschriebenen Methode erhaltenen Ergebnisse wiedergegeben.

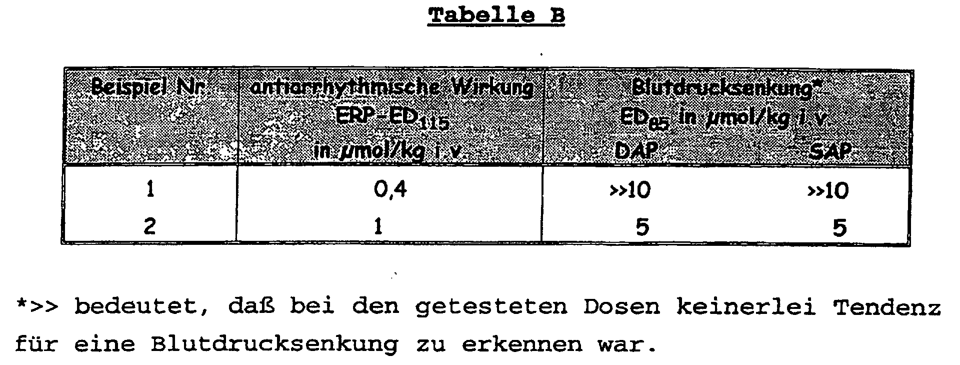

### 3. In vitro Bestimmung der Wirkung der Substanzen auf die funktionelle Refraktärzeit an elektrisch stimulierten Papillarmuskeln aus Meerschweinchenherzen.

Die die Refraktärzeit verlängernde Wirkung der Substanzen läßt sich auch in in vitro Tests durch Bestimmung der funktionellen Refraktärzeit (=FRP) am isolierten Papillarmuskel der rechten Herzkammer von Meerschweinchen nachweisen.

Durch Genickschlag getöteten Meerschweinchen wurde das Herz rasch entnommen und die Papillarmuskeln der rechten Herzkammer wurden in Organbädern befestigt, welche mit temperierter oxygenierter Nährlösung durchströmt wurden. Die Muskelpräparate wurden elektrisch mit einer Frequenz von 3 Hz gereizt. Die Testsubstanzen wurden den Organbädern in steigenden Konzentrationen (kumulativ) zugesetzt. Jeweils 20 Minuten nach Testsubstanzzugabe wurde mittels eines Doppelpulsprotokolls die funktionelle Refraktärzeit bestimmt. Aus den gemessenen Werten wurde jeweils eine kumulative Konzentrations-Wirkungskurve aufgestellt. Aus dieser wurde die Konzentration, bei der eine Verlängerung der FRP um 12 Millisekunden erreicht wurde, als effektive Konzentration (= FRP-EC₊₁₂ₘₛ) berechnet.

In der nachfolgenden Tabelle C werden die mit der vorstehend beschriebenen Methode erhaltenen Ergebnisse wiedergegeben.

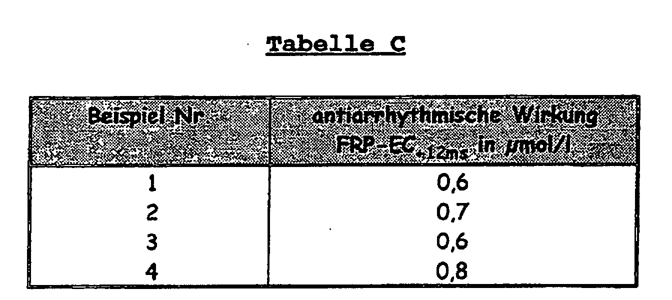

### 4. In vitro Bestimmung der potentiellen Proarrhythmogenität der Substanzen am isoliert perfundierten Kaninchenherzen

Das Ausmaß der potentiellen Proarrhythmogenität von Klasse-III antiarrythmisch wirksamen Substanzen kann anhand der Messung elektrophysiologischer Parameter wie "Instabilität" und "Triangulierung "(s. u.) am monophasischen Aktionspotential, abgeleitet an isoliert perfundierten Kaninchenherzen, eingeschätzt werden. Der nachfolgend angegebene pharmakologische Test wurde analog zu der grundlegend bei L. M. Hondeghem et al., Circulation 103 (2001) 2004-2013 (nachfolgend zitiert als "Hondeghem et al."), in Verbindung mit L. M. Hondeghem, Journal of Cardiovascular Electrophysiology 5(8) (1994) 711-721 (nachfolgend zitiert als "Hondeghem"), beschriebenen Methode durchgeführt.

Durch Genickschlag getöteten Kaninchen wurde das Herz rasch entnommen und sofort in einer Langendorff-Anordnung druckkonstant (80 cm H₂O) mit temperierter oxygenierter Nährlösung perfundiert. Mit Hilfe zweier Stimulationselektroden, die jeweils im Bereich des rechten und linken HIS-Bündelschenkels angeordnet wurden, konnte das Herz mit unterschiedlichen Stimulationsprotokollen stimuliert werden (vgl. "Hondeghem et al."). Zwei weitere Elektroden (eine Ableitelektrode endokardial im Bereich des Septums der linken Herzkammer und eine Referenzelektrode epikardial an der linken Herzkammer) dienten der Ableitung des monphasischen Aktionspotentiales.

Anhand der monophasischen Aktionspotentialdauer bei unterschiedlicher Repolarisation (APD10-90; als "APD10" wird die Aktionspotentialdauer bis zum Eintritt von 10 % der Repolarisation bezeichnet) wurden folgende Parameter als Indikatoren für die Proarrhythmogenität abgeleitet:
(1) Instabilität: Als Instabilität wird die Änderung der APD60 von Herzschlag zu Herzschlag bezeichnet. Unter Kontrollbedingungen beträgt dieser Wert durchschnittlich etwa 7 Millisekunden (= ms). Höhere Abweichungen von diesem Durchschnittswert hin zu längerer Zeitdauer (>7 ms) weisen auf eine erhöhte Eintrittswahrscheinlichkeit für durch die untersuchten Testsubstanzen verursachte Proarrhythmien hin.
(2) Triangulierung: Als Triangulierung wird die Repolarisationszeit in ms von APD30 zu ADP90 bezeichnet. Unter Kontrollbedingungen liegt dieser Wert üblicherweise bei etwa 90 ms. Eine unter dem Einfluß einer Testsubstanz signifikant über diesen Kontrollwert verlängerte Repolarisationszeit deutet auf einen verlangsamten Repolarisationsvorgang hin, was wiederum zu einer erhöhten Rate an Nachpolarisationen (= Proarrhythmien) führen kann.

In der nachfolgenden Tabelle D werden die mit den vorstehend beschriebenen Methoden erhaltenen Ergebnisse an jeweils drei Herzen (n=3) wiedergegeben.

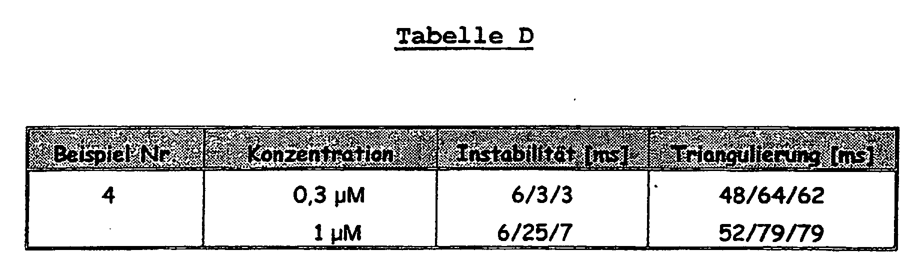

### 5. Blockadewirkung auf den schnellen bzw. langsamen verzögert gleichrichtenden Kaliumstrom ("rapid bzw. slow delayed rectifier potassium current"; "iKr" bzw. "iKs")

Das Wirkprinzip der sogenannten Klasse-III antiarrhythmisch wirksamen Substanzen (nach Vaughan-Williams) beruht auf deren Blockade unterschiedlicher an der Repolarisation des kardialen Aktionspotentiales beteiligter zellulärer Kalium-Auswärtsströme. Dies führt zu einer Verlängerung der kardialen Refraktärzeit, wodurch Herzrhythmusstörungen verhindert werden können. Dabei hängt das proarrhythmogene Risiko von Klasse-III antiarrhythmischen Substanzen davon ab, welcher Kaliumstrom bzw. welche Kombination von Kaliumströmen blockiert wird. Aus der Literatur ist bekannt, daß die selektive iKr-Blockade ein hohes proarrhythmogenes Risiko bergen kann, während der gleichzeitigen Blockade des iKr und des iKs ein deutlich vermindertes proarrhythmogenes Risiko zugeschrieben wird.

Der iKs kann auf die nachfolgend angegebene Weise selektiv gemessen werden: narkotisierten Hunden wird rasch das Herz entnommen und ein Muskelabschnitt der linken Herzkammer wird mit kollagenasehaltiger Lösung über ein arterielles Gefäß perfundiert. Das gut dissoziierte Gewebe wird zerkleinert und die vereinzelten Herzmuskelzellen werden elektrophysiologisch mit der "Ganzzell Patch-clamp"-Methode (vgl. O. P. Hamill et al., Pflügers Archive 391(2) (1981) 85-100) untersucht: zur selektiven Untersuchung der Blockadewirkung auf den iKs werden der Calcium-Einwärtstrom durch Zugabe von 1µM Nisoldipine (= selektiver Blocker des Calcium-Einwärtsstromes), der iKr durch Zugabe von 2µM E-4031 (= selektiver iKr-Blocker) und der schnelle Natrium-Einwärtstrom sowie der transiente Kaliumauswärtstrom durch ein Haltepotential von -40mV blockiert. Der iKs wird sodann anhand der Stromamplitude sofort am Ende eines 5-Sekunden Pulsprotokolles von -40mV Haltepotential auf maximal +50mV Depolarisation ermittelt.

Der iKr (HERG) kann auf die nachfolgend angegebene Weise selektiv gemessen werden: zur Messung des iKr wird eine Zelllinie (Humane embryonale Nierenzellen, HEK293; siehe z. B. American Tissue Culture Collection (= ATCC)-Nr.: CRL-1573) verwendet, die stabil mit dem Gen für den iKr (HERG) transfiziert ist (vgl. Z. Zhou et al., Biophysical Jornal 74(1) (1998) 230 - 241). Da die verwendeten Zellen keine weiteren die Messung störenden Ionenströme besitzen, kann auf entsprechende Zugaben von kanalblockierenden Substanzen verzichtet werden. Der iKr wird anhand der Stromamplitude bei -40 mV Haltepotential sofort nach einem 500-ms Pulsprotokoll von - 75 mV Haltepotential auf maximal +10 mV Depolarisation ermittelt.

Aus den Inhibitionen des entsprechenden Stromes bei unterschiedlichen Substanzkonzentrationen wurde diejenige Substanzkonzentration ermittelt, bei der 50 % des Maximalstromes blockiert ist (IC50 %). In der nachfolgenden Tabelle E werden die mit den vorstehend beschriebenen Methoden erhaltenen Ergebnisse wiedergegeben:

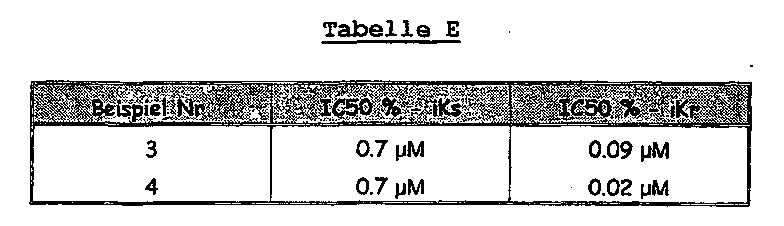

In einem weiteren in vivo Test an narkotisierten Katzen zeigte die Verbindung des Beispieles 4 nach Verabreichung p.o. und i.v. jeweils eine dosisabhängige deutliche und langanhaltende Erhöhung der Flimmerschwelle, welche am Vorhof stärker ausgeprägt war, als an der Herzkammer. Eine derartige atrioselektive Erhöhung der Flimmerschwelle ist ein Indiz dafür, daß die getestete Verbindung ein günstiges Wirkungsprofil mit einem verminderten Proarrhythmogenitäts-Risiko aufweist.

Die vorstehenden Testergebnisse zeigen, daß die Verbindungen der Formel I antiarrhythmische Wirkungen besitzen und die effektive Refraktärzeit des Herzmuskels deutlich verlängern und daß eine effektive blutdrucksenkende Wirkung der Substanzen erst bei Dosen auftritt, die erheblich höher als die für die Refraktärzeitverlängerung effektiven Dosen sind. Die vorstehenden Testergebnisse zeigen auch einen Zusammenhang auf zwischen der überraschend geringen Neigung der erfindungsgemäßen Substanzen zur Entfaltung proarrhythmogener Begleitwirkungen und deren spezifischem Profil der Beeinflussung der unterschiedlichen auswärts gerichteten Kaliumströme in Herzzellen von größeren Säugetieren und Menschen, beispielsweise der Beeinflussung des iKr und des iKs.

Aufgrund ihres vorstehend beschriebenen Wirkungsprofils eignen sich die Substanzen zur Unterdrückung von tachycarden Herzrhythmusstörungen und können zur Prophylaxe und Behandlung von Herzrhythmusstörungen in größeren Säugetieren und Menschen Verwendung finden. Insbesondere sind die Substanzen geeignet, das Auftreten von Tachyarrhythmien, d. h. Arrhythmien, die mit einem Anstieg der Herzfrequenz gekoppelt sind, zu verhindern.

Die zu verwendenden Dosen könnnen individuell verschieden sein und variieren naturgemäß je nach Art des zu behandelnden Zustandes, der verwendeten Substanz und der Applikationsform. Im allgemeinen eignen sich jedoch für Applikationen an größeren Säugetieren, insbesondere Menschen, Arzneiformen mit einem Wirkstoffgehalt von 0,5 bis 100, insbesondere 1 bis 25, mg pro Einzeldosis.

Als Heilmittel können die Verbindungen der Formel I mit üblichen pharmazeutischen Hilfsstoffen in galenischen Zubereitungen wie z. B. Tabletten, Kapseln, Suppositorien oder Lösungen enthalten sein. Diese galenischen Zubereitungen können nach an sich bekannten Methoden hergestellt werden unter Verwendung üblicher fester oder flüssiger Träger wie z. B. Milchzucker, Stärke oder Talkum oder flüssigen Paraffinen und/oder unter Verwendung von üblichen pharmazeutischen Hilfsstoffen, beispielsweise Tablettensprengmitteln, Lösungsvermittlern oder Konservierungsmitteln.

Die nachfolgenden Beipiele sollen die Erfindung näher erläutern, jedoch deren Umfang in keiner Weise beschränken.

### Beispiel 1:

### 7-(n-Butyl)-3-(2,4-dinitrophenyl)-9,9-dimethyl-3,7-diazabicyclo[3,3,1]nonan

Zu einer Lösung von 4,2 g 2,4-Dinitrofluorbenzol in 40 ml Aceton wurde unter Rühren eine Lösung von 4,7 g 7-(n-Butyl)-9,9-dimethyl-3,7-diazabicyclo[3,3,1]nonan in 10 ml Aceton zugetropft. Das Reaktionsgemisch wurde bei Raumtemperatur weitergerührt und über Nacht stehen gelassen. Anschließend wurde Aceton abgezogen und der Rückstand mit wäßriger Zitronensäurelösung versetzt und diese Mischung zweimal mit Essigsäureethylester extrahiert. Zur weiteren Aufarbeitung wurde der wäßrige Rückstand mit verdünnter Natronlauge alkalisiert. Dieses Gemisch wurde erneut zweimal mit Essigsäureethylester extrahiert. Die vereinigten organischen Phasen wurden mit Magnesiumsulfat getrocknet und eingegengt. Der Rückstand wurde aus Ether/Hexan umkristallisiert. Es wurden 5,5 g 7-(n-Butyl)-3-(2,4-dinitrophenyl)-9,9-dimethyl-3,7-diazabicyclo-[3,3,1]nonan mit einem Schmelzpunkt von 119 °C erhalten.

0,61 g der Titelverbindung wurden in 10 ml Isopropanol unter Erwärmen in einem Ölbad gelöst. Zu der Lösung wurden 0,7 ml einer 3,8 n isopropanolischen Salzsäurelösung gegeben. Dann wurde das Reaktionsgemisch abkühlen gelassen und die gebildeten Kristalle wurden abfiltriert. Es wurden 0,5 g des Hydrochlorids der Titelverbindung mit einem Schmelzpunkt von 136 - 138 °C erhalten.

Nach dem in dem vorstehenden Beispiel beschriebenen oder nach hierzu analogen Verfahren können auch die in der folgenden Tabelle F angeführten Verbindungen der Formel I hergestellt werden.

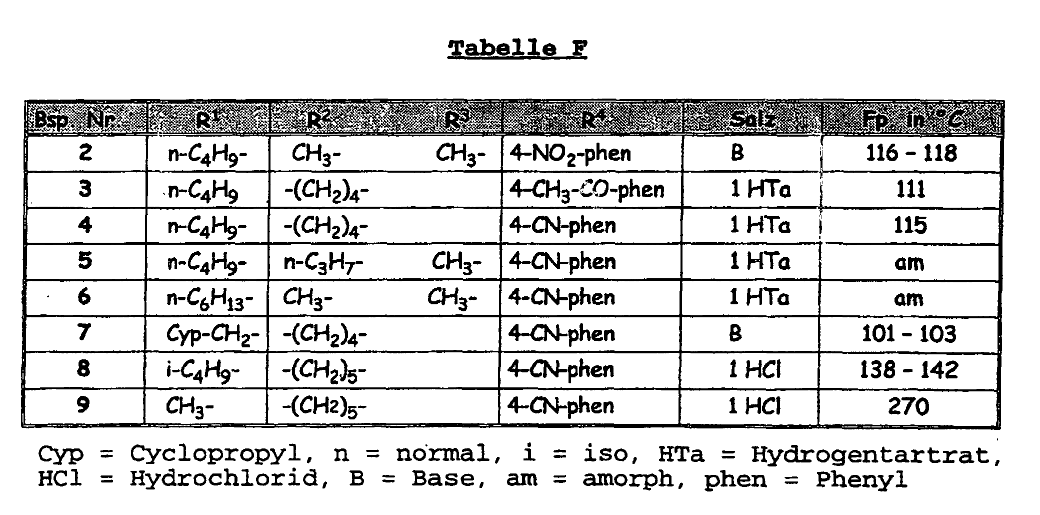

### Beispiel I:

### 7-(n-Butyl)-3-(4-cyanophenyl)-9,9-tetramethylen-3,7-diazabicyclo[3,3,1]nonan-hydrogentartrat enthaltende Tabletten

Man stellte Tabletten in folgender Zusammensetzung pro Tablette her:

| | |
|---|---|
| 7-(n-Butyl)-3-(4-cyanophenyl)-9,9-tetramethylen-3,7-diazabicyclo[3,3,1]nonan-hydrogentartrat | 20 mg |
| Maisstärke | 60 mg |
| Milchzucker | 135 mg |
| Gelatine (als 10%ige Lösung) | 6 mg |

Der Wirkstoff, die Maisstärke und der Milchzucker wurden mit der 10%igen Gelatine-Lösung eingedickt. Die Paste wurde zerkleinert, und das entstehende Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

## Patentansprüche

1. Verbindungen der allgemeinen Formel I 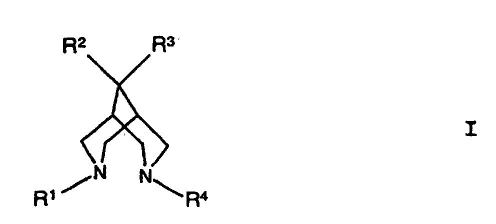 worin
R¹ eine Alkylgruppe mit 1 - 6 Kohlenstoffatomen oder eine Cycloalkylalkylgruppe mit 4 - 7 Kohlenstoffatomen bedeutet,
R² niederes (C₁-C₄) Alkyl bedeutet und
R³ niederes (C₁-C₄) Alkyl bedeutet oder
R² und R³ gemeinsam eine Alkylenkette mit 3 - 6 Kohlenstoffatomen bilden,
R⁴ für einen in ortho- oder para-Stellung durch Nitro, Cyano oder niederes (C₂-C₅) Alkanoyl monosubstituierten oder in ortho- und para-Stellung durch Nitro disubstituierten Phenylrest steht, und
deren physiologisch verträgliche Säureadditionssalze.

2. Verbindungen gemäß Anspruch 1, worin R⁴ einen in para-Stellung durch Cyano oder niederes (C₂-C₅) Alkanoyl monosubstituierten Phenylrest bedeutet.

3. Verbindungen gemäß einem der vorstehenden Ansprüche, worin R¹ eine Alkylgruppe mit 3 - 5 Kohlenstoffatomen bedeutet und R² und R³ gemeinsam eine Alkylenkette mit 3 bis 6 Kohlenstoffatomen bilden.

4. 7-(n-Butyl)-3-(4-cyanophenyl)-9,9-tetramethylen-3,7-diazabicyclo[3,3,1]nonan gemäß Anspruch 1.

5. 7-(n-Butyl)-3-(4-acetylphenyl)-9,9-tetramethylen-3,7-diazabicyclo[3,3,1]nonan gemäß Anspruch 1.

6. Arzneimittel, **dadurch gekennzeichnet, daß** sie eine pharmakologisch wirksame Menge einer Verbindung gemäß Anspruch 1 und pharmazeutische Hilfs- und/oder Trägerstoffe enthalten.

7. Verwendung von Verbindungen gemäß Anspruch 1 zur Herstellung pharmazeutischer Zubereitungen für die Behandlung und/oder Prophylaxe von Herzrhythmusstörungen.

8. Verfahren zur Herstellung von Verbindungen der allgemeinen Formel I 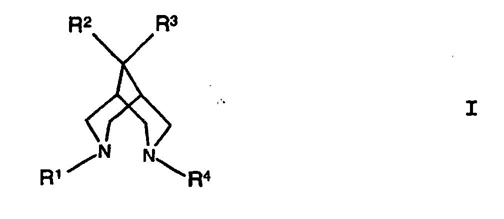 worin
R¹ eine Alkylgruppe mit 1 - 6 Kohlenstoffatomen oder eine Cycloalkylalkylgruppe mit 4 - 7 Kohlenstoffatomen bedeutet,
R² niederes (C₁-C₄) Alkyl bedeutet und
R³ niederes (C₁-C₄) Alkyl bedeutet oder
R² und R³ gemeinsam eine Alkylenkette mit 3 - 6 Kohlenstoffatomen bilden,
R⁴ für einen in ortho- oder para-Stellung durch Nitro, Cyano oder niederes (C₂-C₅) Alkanoyl monosubstituierten oder in ortho- und para-Stellung durch Nitro disubstituierten Phenylrest steht, und
deren physiologisch verträglichen Säureadditionssalzen, **dadurch gekennzeichnet, daß** man Verbindungen der allgemeinen Formel II 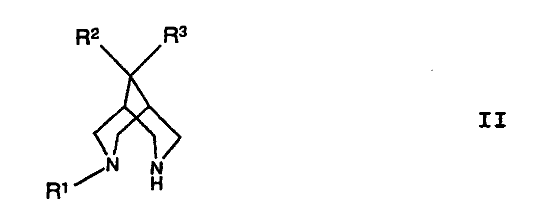 worin R¹, R² und R³ obige Bedeutung besitzen, mit Verbindungen der allgemeinen Formel III
R⁴ - X III
worin R⁴ obige Bedeutung besitzt und X Halogen bedeutet, umsetzt, und gegebenenfalls freie Verbindungen der Formel I in ihre Säureadditionsalze überführt oder die Säureadditionssalze in die freien Verbindungen der Formel I überführt.

## Claims

1. Compounds of the general formula I, 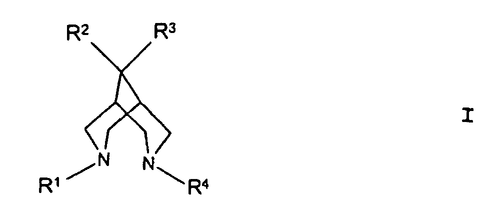 wherein
R¹ is an alkyl group with 1 - 6 carbon atoms or a cycloalkylalkyl group with 4 - 7 carbon atoms,
R² is lower (C₁-C₄) alkyl and
R³ is lower (C₁-C₄) alkyl or
R² and R³ together form an alkylene chain with 3 - 6 carbon atoms,
R⁴ stands for a phenyl radical monosubstituted in the ortho or para position by nitro, cyano or lower (C₂-C₅) alkanoyl or disubstituted in the ortho and para position by nitro, and
their physiologically compatible acid addition salts.

2. Compounds according to Claim 1, wherein R⁴ is a phenyl radical monosubstituted in the para position by cyano or lower (C₂-C₅) alkanoyl.

3. Compounds according to one of the preceding claims, wherein R¹ is an alkyl group with 3 - 5 carbon atoms and R² and R³ together form an alkylene chain with 3 to 6 carbon atoms.

4. 7-(n-butyl)-3-(4-cyanophenyl)-9,9-tetramethylene-3,7-diazabicyclo[3.3.1]nonane according to Claim 1.

5. 7-(n-butyl)-3-(4-acetylphenyl)-9,9-tetramethylene-3,7-diazabicyclo[3.3.1]nonane according to Claim 1.

6. Medicaments, **characterised in that** they contain a pharmacologically active quantity of a compound according to Claim 1 and pharmaceutical auxiliaries and/or carriers.

7. The use of compounds according to Claim 1 for the preparation of pharmaceutical preparations for the treatment and/or prophylaxis of cardiac arrhythmias.

8. A process for the preparation of compounds of the general formula I 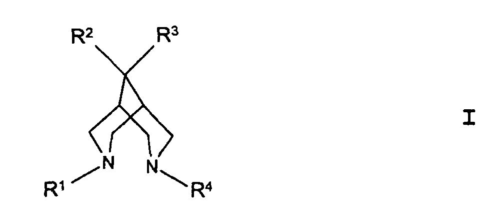 wherein
R¹ is an alkyl group with 1 - 6 carbon atoms or a cycloalkylalkyl group with 4 - 7 carbon atoms,
R² is lower (C₁-C₄) alkyl and
R³ is lower (C₁-C₄) alkyl or
R² and R³ together form an alkylene chain with 3 - 6 carbon atoms,
R⁴ stands for a phenyl radical monosubstituted in the ortho or para position by nitro, cyano or lower (C₂-C₅) alkanoyl or disubstituted in the ortho and para position by nitro, and
the physiologically compatible acid addition salts thereof, **characterised in that** compounds of the general formula II 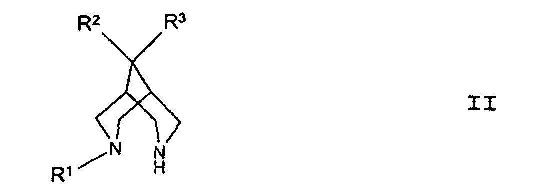 wherein R¹, R² and R³ have the above meaning, are reacted with compounds of the general formula III
R⁴ - X III
wherein R⁴ has the above meaning and X is halogen,
and optionally free compounds of Formula I are converted into their acid addition salts or the acid addition salts are converted into the free compounds of Formula I.

## Revendications

1. Composés de formule générale I 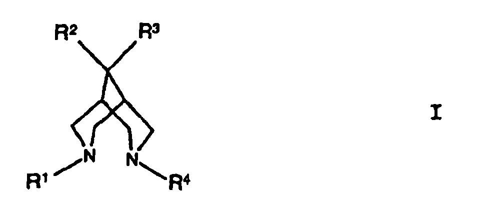 dans laquelle
R¹ est un groupe alkyle comportant 1 à 6 atomes de carbone ou un groupe cycloalkylalkyle comportant 4 à 7 atomes de carbone,
R² est un alkyle inférieur en (C₁-C₄) et
R³ est un alkyle inférieur en (C₁-C₄) ou
R² et R³ forment conjointement une chaîne alkylène comportant 3 à 6 atomes de carbone,
R⁴ représente un radical phényle monosubstitué en position ortho ou para par un groupe nitro, cyano ou alkanoyle inférieur en (C₂-C₅), ou disubstitué en position ortho et para par un groupe nitro, et
leurs sels d'addition acides physiologiquement compatibles.

2. Composés selon la revendication 1, où R⁴ représente un radical phényle monosubstitué en position para par un groupe cyano ou par un alcanoyle inférieur en (C₂-C₅).

3. Composés selon l'une des revendications précédentes, où R¹ représente un groupe alkyle comportant 3 à 5 atomes de carbone et R² et R³ forment conjointement une chaîne alkylène comportant 3 à 6 atomes de carbone.

4. 7-(n-butyl)-3-(4-cyanophényl)-9,9-tétraméthylène-3,7-diazabicyclo [3,3,1]nonane selon la revendication 1.

5. 7-(n-butyl)-3-(4-acétylphényl)-9,9-tétraméthylène-3,7-diazabicyclo [3,3,1]nonane selon la revendication 1.

6. Médicaments **caractérisés en ce qu'**ils contiennent une quantité efficace sur le plan pharmacologique d'un composé selon la revendication 1 et des auxiliaires et/ou des véhicules pharmaceutiques.

7. Utilisation de composés selon la revendication 1 pour fabriquer des préparations pharmaceutiques destinées au traitement et/ou à la prophylaxie des irrégularités du rythme cardiaque.

8. Procédé de préparation de composés de formule générale I 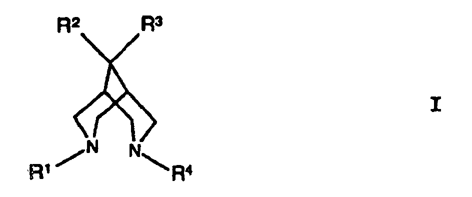 dans laquelle
R¹ est un groupe alkyle comportant 1 à 6 atomes de carbone ou un groupe cycloalkylalkyle comportant 4 à 7 atomes de carbone,
R² est un alkyle inférieur en (C₁-C₄) et
R³ est un alkyle inférieur en (C₁-C₄) ou
R² et R³ forment conjointement une chaîne alkylène comportant 3 à 6 atomes de carbone,
R⁴ représente un radical phényle monosubstitué en position ortho ou para par un groupe nitro, cyano ou alcanoyle inférieur en (C₂-C₅), ou disubstitué en position ortho et para par un groupe nitro, et
leurs sels d'addition acides physiologiquement compatibles, **caractérisé en ce que** l'on fait réagir des composés de formule générale II 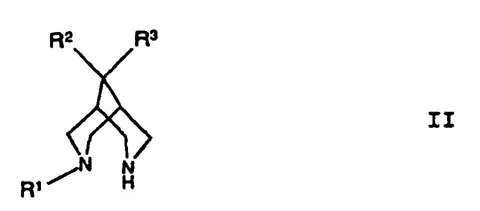 dans laquelle R¹, R² et R³ ont les significations données précédemment, avec des composés de formule générale III
R⁴-X III
dans laquelle R⁴ a la signification donnée précédemment et X représente un halogène, et que l'on transforme éventuellement les composés libres de formule I en leurs sels d'addition acides ou que l'on transforme les sels d'addition acides en composés libres de formule I.
